# EUROPÄISCHE PATENTSCHRIFT

(11) **EP 0 784 048 B1**
(45) Veröffentlichungstag und Bekanntmachung des Hinweises auf die Patenterteilung: **31.10.2001**
(21) Anmeldenummer: 97100031.0
(22) Anmeldetag: 03.01.1997
(51) Int. Cl.: C07C 68/08, C07C 69/96

(54) **Verfahren zur Reinigung von Kohlensäurediarylester**
Process for purifying diaryl esters of carbonic acid
Procédé de purification d'esters diaryliques d'acide carbonique

(30) Priorität: 10.01.1996 DE 19600631
(43) Veröffentlichungstag der Anmeldung: 16.07.1997
(73) Patentinhaber: BAYER AG, 51368 Leverkusen (DE)
(72) Erfinder: Kühling, Steffen, Dr., 40670 Meerbusch (DE); Hallenberger, Kaspar, Dipl.-Ing., 51375 Leverkussen (DE); Ooms, Pieter, Dr., 47800 Krefeld (DE); Buysch, Hans-Josef, Dr., 47809 Krefeld (DE); Jeromin, Günther, Dr., 47802 Krefeld (DE)

(56) Entgegenhaltungen:
- EP-A- 0 633 241

## Beschreibung

Gegenstand der vorliegenden Erfindung ist ein destillatives Reinigungsverfahren von Kohlensäurediarylestern durch kontinuierliche Destillation, bei dem der Kohlensäurediarylester bei Sumpftemperaturen >150°C destilliert und im Seitenstrom aus der Dampfphase einer Kolonne abgefaßt wird.

Bei der Herstellung von Polycarbonaten aus Kohlensäurediarylestern und Diphenolen nach dem Umesterungsverfahren ist für eine gute Qualität des resultierenden Polycarbonats und für eine problemlose Reaktionsführung und Katalyse des Prozesses eine gleichbleibend hohe Qualität des Kohlensäurediarylesters von extremer Wichtigkeit. Die Reinigung der Kohlensäurediarylester, die durch Reaktion von Monophenolen und Phosgen in einem Lösungsmittel in Gegenwart von Alkali in der Phasengrenzfläche hergestellt wurden, durch Destillation ist in der Encyclopedia of Polymer Science, Vol. 10 (1969), Chemistry and Physics of Polycarbonates, Polymer Reviews, H. Schnell, Vol. 9, John Wiley and Sons, Inc. (1964), S. 50/51 beschrieben.

In EP-A 0 633 241 wird ein Reinigungsverfahren für Diarylcarbonate offenbart, welches aus einem Waschvorgang und anschließende Flash-Destillation mit Entnehmen dreier Fraktionen "über Kopf" besteht. Dieses Verfahren ist jedoch nicht für eine kontinuierliche Fahrweise geeignet.

Es wurde nun gefunden, daß durch ein gezieltes kontinuierliches Destillationsverfahren die Kohlensäurediarylester eine verbesserte Qualität besitzen, die sie besonders geeignet macht für die Polycarbonatherstellung nach dem Schmelzumesterungsprozeß.

Kohlensäurediester im Sinne vorliegender Erfindung sind Di-C₆-C₂₀-Arylester, vorzugsweise die Diester von Phenol oder alkylsubstituierten Phenolen, also Diphenylcarbonat oder z.B. Dikresylcarbonat, bevorzugt jedoch Diphenylcarbonat.

Die für eine Reinigung nach dem erfindungsgemäßen Verfahren geeigneten Kohlensäurediester können durch unterschiedliche Verfahren hergestellt worden sein, beispielsweise mittels des Phasengrenzflächenverfahrens (Encyclopedia of Polymer Science, Vol. 10 (1969), Chemistry and Physics of Polycarbonates, Polymer Reviews, H. Schnell, Vol. 9, John Wiley and Sons, Inc. (1964), des Direktverfahrens aus Monophenol und Carbonyldihalogenid (z.B. in EP-A 483 632 beschrieben) des carbonyldihalogenidfreien Direktverfahrens aus Kohlenmonoxid und Monophenol (z.B. DE-OS 27 38 437) und des Umesterungsverfahrens von Dialkylcarbonaten, besonders Dimethylcarbonat und Monophenolen (z.B. JP-O-291 257, JP-O-93 660). Bevorzugt sind die nach dem Umesterungsverfahren oder dem Direktverfahren aus Monophenol und Carbonyldihalogenid hergestellten Diester, da sich das Reinigungsverfahren, neben der Abtrennung von anorganischen Verunreinigungen insbesondere zur Reinigung des Kohlensäurediarylesters von organischen Nebenprodukten eignet. Dies können im Falle des Diphenylcarbonats Chlorameisensäurephenylester, 2-Phenoxybenzoesäure, chlorierte Arylcarbonate, Xanthon, insbesondere aber Salicylsäurephenylester sein. Die Abtrennung von Salicylsäurephenylester ist besonders problematisch, da die Siedepunkte des Diphenylcarbonats und des Salicylsäurephenylesters nahezu gleich sind.

Die beispielsweise nach dem in EP-A 483 632 beschriebenem Verfahren hergestellten Kohlensäurephenylester können je nach Versuchsbedingungen 1200 bis 7000 ppm Salicylsäurephenylester enthalten.

Überraschend wurde nun gefunden, daß trotz der sehr kleinen Siedepunktdifferenz bei einer kontinuierlichen Destillation durch Seitenstromentnahme des Kohlensäurediarylesters der Gehalt an organischen Bestandteilen, insbesondere des Salicylsäurephenylesters, auf <1000 ppm, bevorzugt <800 ppm, besonders bevorzugt <500 ppm, ganz besonders bevorzugt <300 ppm reduziert werden kann.

Die Entnahme eines dampfförmigen Seitenstroms ist literaturbekannt und beispielsweise in der Patentschrift DE 4 214 738 beschrieben.

Gegenstand der vorliegenden Erfindung ist demnach ein Verfahren zur destillativen Reinigung von aromatischen Kohlensäurediestern, dadurch gekennzeichnet, dass das zu reinigende Produkt über einen Zulauf, der zwischen dem Sumpf und dem Kopf einer Destillationseinheit angebracht ist, kontinuierlich in eine Destillationseinheit eingebracht wird und das Produkt aus der Dampfphase über eine Seitenstrom zwischen Zulauf und Sumpf kontinuierlich entnommen wird.

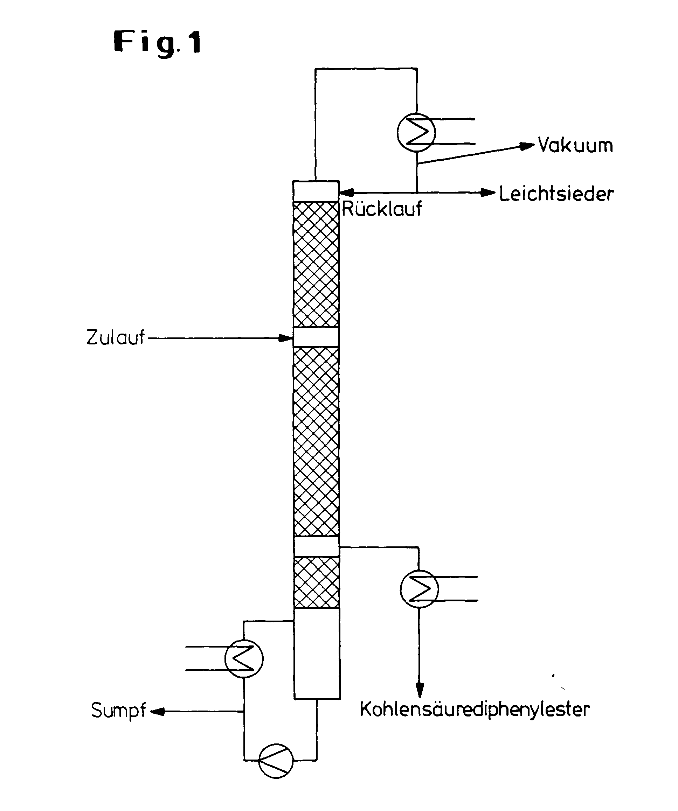

Im erfindungsgemäßen Verfahren wird die Destillation kontinuierlich so geführt, daß die Sumpftemperatur bei der Destillation >150°C bis 310°C, bevorzugt >160 bis 230°C beträgt. Dazu wird in der Kolonne ein Druck zwischen 1 und 1000 mbar, bevorzugt zwischen 5 und 100 mbar eingestellt. Die mittlere Verweilzeit des Rohkohlensäurediarylesters beträgt je nach Prozeßführung zwischen 1 und 10 Minuten. Die mittlere Verweilzeit des Sumpfproduktes in der Destillationsapparatur beträgt je nach Verdampferart zwischen 1 min und mehreren Tagen unter Betriebsbedingungen. Die anfallenden Produktströme werden über barometrische Leitungsführungen oder durch geeignete Pumpen der Anlage entnommen. Die produktberührten Leitungen und Aggregate werden auf Temperaturen oberhalb der Produktschmelzpunkte beheizt. Zur Erzielung der notwendigen Trennleistung ist die Kolonne mit geordneter Feinvakuumpackung, geordneter Blechpackung oder ungeordneten Füllkörpern zu füllen. Zur Trennung werden 5 bis 15 theoretische Stufen bei Rücklaufverhältnissen von 0,5 bis 10 eingesetzt. Das Prinzip der Destillation ist in Fig. 1 dargestellt.

Die so gereinigten Kohlensäurediester zeichnen sich aus durch hohe Reinheit (GC>99,9%) und extrem gutes Umesterungsverhalten (1*10⁻⁴ mol-% Katalysator, z.B. NaOH, reichen für den Start der Umesterung aus), so daß ein Polycarbonat in exzellenter Qualität hergestellt werden kann.

Die Herstellung von aromatischen Oligo-/Polycarbonaten nach dem Schmelzeumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-P 5 340 905 vorbeschrieben.

### Beispiele

### Beispiel 1

Für die Destillation des nach dem Direktphosgenierverfahren hergestellten Diphenylcarbonat wurde eine Kolonne mit 0,5 m Verstärkungsteil und 1 m Abtriebsteil aus geordneter Feinvakuumpackung mit einem Innendurchmesser von 50 mm verwendet (vgl. Fig. 1). Als Kolonnenkopf wurde ein Dampfteiler eingesetzt, der für die Kondensation von sublimierenden Produkten modifiziert war. Die Kondensation erfolgte mit Wasser bei 80°C. In der Regel wurde bei einem Kopfdruck von 12 mbar gefahren, gemessen hinter der Kondensation. Die Kopftemperatur lag bei 155°C, die Temperatur an der Seitenentnahme betrug 160°C. Das Rücklaufverhältnis wurde auf einen Wert von 5 eingestellt. Der adiabate Mantel der Kolonne wurde mit der Innentemperatur abgeglichen. Bei einer Zulaufinenge von 600 g/h wurde eine Destillatmenge von 30 g/h Leichtsieder über Kopf abgezogen. Unterhalb des Abtriebsteils wurden im Seitenstrom 570 g/h Kohlensäurediarylester entnommen. Die so erzielte GC-Produkteinheit betrug 99,96 %; dabei konnte der Salicylsäurephenylester von 3000 ppm im Rohprodukt auf Konzentrationen von 200 ppm abgereinigt werden.

Das so erhaltene Diphenylcarbonat wird einem Schmelze-PC-Eignungstest unterzogen. Das Umesterungsverhalten und somit die Eignung des Kohlensäurediphenylesters für die Schmelzeumesterung wird durch das Starttemperatur-Verfahren bestimmt. Dabei wird das Reaktionsgemisch aus 17,1 g (0,075 mol) 2,2-Bis-(4-hydroxyphenyl)-propan und 17,0 g (0,07945 mol) des zu prüfenden Kohlensäurediphenylester mit 0,0001 mol-% NaOH (bezogen auf BPA) (1 %ige wäßrige Lösung) katalysiert und in einem 100 ml-Kolben mit Brücke und Thermometer in ein auf 270°C vorgeheiztes Ölbad eingesetzt. Die Starttemperatur und die Zeit, die das Eduktgemisch zur Umesterung und somit zum Destillationsbeginn (Phenolabspaltung) benötigt, werden zum Vergleich notiert.

Für ein gutes Umesterungsverhalten ist es notwendig, daß die Umesterung bereits mit geringen Konzentrationen an Katalysator einsetzt. Dies ist dann gegeben, wenn bei dem Starttemperaturverfahren die Sumpftemperatur <260°C bleibt und das erste abgespaltene Phenol nach <20 min destilliert.

Das nach dem obengenannten Verfahren gereinigte DPC zeigt nach 14 min und einer Sumpftemperatur von 255°C die erste Destillation.

### Beispiel 2

Die Destillation wurde in derselben Apparatur wie in Beispiel 1 durchgeführt, nur wurde bei einer Kolonnentemperatur von 210°C und entsprechend höherem Druck destilliert. Bei gleicher Mengenbilanz und Zulaufkonzentration konnte der Salicylsäurephenylester von 3000 ppm auf Konzentrationen von 50 ppm abgereinigt werden. Das destillierte DPC zeigt nach 13 min und einer Sumpftemperatur von 253°C die erste Destillation.

## Patentansprüche

1. Verfahren zur destillativen Reinigung von aromatischen Kohlensäurediestern, **dadurch gekennzeichnet, dass** das zu reinigende Produkt über einen Zulauf, der zwischen dem Sumpf und dem Kopf einer Destillationseinheit angebracht ist, kontinuierlich in eine Destillationseinheit eingebracht wird und das Produkt aus dem Dampfphase über eine Seitenstrom zwischen Zulauf und Sumpf kontinuierlich entnommen wird.

2. Verfharen gemäß Anspruch 1, **dadurch gekennzeichnet, dass** die Destillation bei einer Sumpftemperatur größer 150°C durchgeführt wird.

3. Verfahren gemäß mindestens einem der obigen Ansprüche, **dadurch gekennzeichnet, dass** die Destillation bei einem Druck zwischen 1 und 1000 mbar durchgeführtwird.

4. Verfahren gemäß mindestens einem der obigen Ansprüche, **dadurch gekennzeichnet, dass** der eingesetzte Kohlensäurediester Diphenylcarbonat ist.

5. Verfahren gemäß mindestens einem der obigen Ansprüche, **dadurch gekennzeichnet, dass** die produktberührten Leitungen und Aggregate auf Temperaturen oberhalb der Produktschmelzpunkte beheizt werden.

6. Verfahren gemäß mindestens einem der obigen Ansprüche, **dadurch gekennzeichnet, dass** zur Trennung in der Destillationseinheit 5 bis 15 theoretische Stufen bei Rücklaufverhältnissen von 0,5 bis 10 eingesetzt werden.

## Claims

1. Process for the purification by distillation of aromatic carbonic acid diesters, **characterised in that** the product to be purified is introduced continuously into a distillation unit via an inlet, which is provided between the bottom and the top of a distillation unit, and the product is removed continuously from the vapour phase via a side stream between the inlet and the bottom.

2. Process according to claim 1, **characterised in that** the distillation is carried out at a bottom temperature greater than 150°C.

3. Process according to at least one of the above claims, **characterised in that** the distillation is carried out at a pressure of between 1 and 1000 mbar.

4. Process according to at least one of the above claims, **characterised in that** the carbonic acid diester used is diphenyl carbonate.

5. Process according to at least one of the above claims, **characterised in that** the ducts and units with which the product comes into contact are heated to temperatures above the melting point of the product.

6. Process according to at least one of the above claims, **characterised in that**, for the purpose of the separation in the distillation unit, 5 to 15 theoretical steps are used at reflux ratios of 0.5 to 10.

## Revendications

1. Procédé de purification par distillation de diesters aromatiques de l'acide carbonique, **caractérisé en ce que** le produit à purifier est introduit en continu dans une unité de distillation, par une admission qui est disposée entre le réservoir et la tête d'une unité de distillation et **en ce que** le produit est prélevé en continu depuis la phase vapeur par un flux de soutirage latéral entre l'admission et le réservoir.

2. Procédé suivant la revendication 1, **caractérisé en ce que** la distillation est réalisée à une température du réservoir supérieure à 150°C.

3. Procédé suivant au moins l'une des revendications précédentes, **caractérisé en ce que** la distillation est réalisée à une pression comprise dans l'intervalle allant de 1 à 1000 mbar.

4. Procédé suivant au moins l'une des revendications précédentes, **caractérisé en ce que** le diester de l'acide carbonique mis en oeuvre est le carbonate de diphényle.

5. Procédé suivant au moins l'une des revendications précédentes, **caractérisé en ce que** les conduites et appareillages convoyant le produit sont chauffés à une température supérieure au point de fusion du produit.

6. Procédé suivant au moins l'une des revendications précédentes, **caractérisé en ce que** pour la séparation dans l'unité de distillation, on met en oeuvre 5 à 15 plateaux théoriques pour un taux de retour situé dans l'intervalle allant de 0,5 à 10.
